# FASCICULE DE BREVET EUROPEEN

(11) **EP 0 811 071 B1**
(45) Date de publication et mention de la délivrance du brevet: **16.01.2002**
(21) Numéro de dépôt: 96904158.1
(22) Date de dépôt: 16.02.1996
(51) Int. Cl.: C12N 15/85, C12N 15/67, A61K 31/70, C12N 1/21, C12N 15/11

(54) **SEQUENCES AMPLIFICATRICES DERIVEES DU GENE DE LA DESMINE, VECTEURS PORTANT CES SEQUENCES ET LEURS UTILISATIONS POUR LA PRODUCTION DE PROTEINES**
VOM DESMINGEN ABSTAMMENDE AMPLIFIZIERUNGSSEQUENZEN, VEKTOREN, DIE DIESE ENTHALTEN UND ANWENDUNGEN DERSELBEN ZUR HERSTELLUNG VON PROTEINEN
AMPLIFIER SEQUENCES DERIVED FROM THE DESMIN GENE, VECTORS COMPRISING SAME AND USES THEREOF FOR PRODUCING PROTEINS

(30) Priorité: 20.02.1995 FR 9501937
(43) Date de publication de la demande: 10.12.1997
(73) Titulaire: INSTITUT PASTEUR, 75724 Paris Cédex 15 (FR); UNIVERSITE PARIS VII, F-75251 Paris Cedex 05 (FR)
(72) Inventeur: PAULIN, Denise, F-94300 Vincennes (FR); LI, Zhen, Lin, 92130 ISSY LES MOULINEAUX (FR)
(74) Mandataire: Phélip, Bruno
(86) Numéro de dépôt international: FR9600261
(87) Numéro de publication internationale: WO9626284

(56) Documents cités:
- EMBO (EUROPEAN MOLECULAR BIOLOGY ORGANIZATION) JOURNAL 13 (15). 3580-3589, 1 Août 1994, XP002006941 LI, H. ET AL.: "An E box in the desmin promoter cooperates with the E box and MEF-2 sites of a distal enhancer to direct muscle -specific transcription."
- J BIOL CHEM 268 (14). 1993. 10403-10415, XP002006942 LI, Z. ET AL.: "DIFFERENT FACTORS INTERACT WITH MYOBLAST -SPECIFIC AND MYOTUBE -SPECIFIC ENHANCER REGIONS OF THE HUMAN DESMIN GENE." cité dans la demande
- NUCLEIC ACIDS RES 21 (2). 1993. 335-343, XP002006943 LI, H. ET AL.: "REGULATION OF THE MOUSE DESMIN GENE TRANSACTIVATION BY MYOD MYOGENIN MRF4 AND MYF5."
- DEVELOPMENT (CAMB) 117 (3). 1993. 947-959, XP000574732 LI, Z. ET AL.: "DESMIN SEQUENCE ELEMENTS REGULATING SKELETAL MUSCLE -SPECIFIC EXPRESSION IN TRANSGENIC MICE." cité dans la demande

## Description

La présente invention concerne des séquences amplificatrices et des vecteurs contenant ces séquences ainsi que l'utilisation de ces vecteurs dans des compositions pour l'expression de séquences nucléotidiques dans ces cellules transfectées, ainsi que les applications thérapeutiques et vaccinales de ces produits.

Le gène de la desmine, l'une des premières protéines musculaires à être détectée lors du développement de l'embryon chez les mammifères, a été séquencé en 1989 par Li et al. (Gene, 78, 243-254).

Ultérieurement, une séquence amplificatrice de 280 paires de bases, située entre les nucléotides -693 et -973 en amont du site d'initiation de la transcription, a été décrite (Li et Paulin, 1991, J. Biol. Chem., 266,6562-6570).

Cette séquence amplificatrice a été mise en évidence en fabriquant une série de constructions plasmidiques portant le gène bactérien codant pour la chloramphénicol acétyle transférase (CAT) puis en les introduisant dans des cellules de souris myogéniques ou non myogéniques. Les auteurs montrent que cette région amplificatrice de 280 paires de bases peut activer aussi bien les promoteurs homologues qu'hétérologues, quelle que soit leur orientation, leur position ou leur distance par rapport à cette séquence.

L'étude de cette séquence a été poursuivie (Li et al., 1993, J. Biol. Chem., 268, 10.403-10.415) et a montré que cette séquence contenait deux régions: l'une active dans les myotubes, et l'autre active dans les myoblastes.

La partie active dans les myotubes est située entre les positions - 973 et - 848, c'est-à-dire dans une séquence de 125 nucléotides.

A l'intérieur de cette séquence, une région comprise entre les positions - 910 et - 870 est protégée de l'action d'une DNase. Cette région contient des sites de liaison des facteurs MEF2 et MyoD1. Aucune activité spécifique n'est indiquée pour cette région de 40 nucléotides. Notamment aucune expérience n'est effectuée avec des constructions contenant cette région isolée. Il est seulement mentionné que les sites MyoD1 et MEF2 sont nécessaires pour avoir une activité amplificatrice complète dans les myotubes.

L'effet amplificatif de la séquence en amont du gène de la desmine a été aussi testé dans une souris transgénique (Li et al., 1993, Development, 117, 947-959). Un fragment de lkb contenant la séquence de régulation en amont du gène de la desmine est lié à un gène rapporteur codant pour la β-galactosidase d'Escherichia coli. Les auteurs montrent que l'activité amplificatrice du promoteur de la desmine est très importante, l'activité β-galactosidase étant très facilement détectée dans des coupes tissulaires.

Il ressort de l'état de la technique analysé ci-dessus que l'on connaissait les propriétés amplificatrices de certaines parties en amont du gène humain de la desmine. Par contre, les régions responsables de cette activité amplificatrice n'étaient pas identifiées.

Il n'était donc pas possible de les modifier avec succès en vue d'améliorer leur performance quant à l'expression de gènes ou de séquences nucléotidiques correspondant en tout ou partie à des c-DNA (ADN complémentaires) codant pour des protéines d'intérêt ou des fragments de ces protéines porteurs d'épitopes capables d'induire des anticorps protecteurs ou des anticorps reconnaissant lesdits épitopes. Des fragments de c-DNA ou d'ADN génomique ont de manière préférentielle une longueur comprise entre 30 paires de bases et 2 kb. Ils peuvent être préparés par synthèse chimique ou par coupure de l'ADN, extrait des cellules ou des microorganismes choisis selon des techniques connues de l'homme du métier, par des enzymes de restriction.

Il est connu que l'introduction d'ADN dans ces cellules eucaryotes selon les techniques décrites notamment dans le brevet WO-90.11.092 ne permet pas d'obtenir une expression prolongée et de haut niveau desdits ADN introduits sous forme de plasmide.

La présente invention apporte une amélioration du niveau d'expression de matériel génétique introduit dans les cellules transfectées par utilisation de constructions mettant en oeuvre tout ou partie des séquences activatrices identifiées pour le gène de la desmine et utilisables pour l'expression de tout autre gène ou matériel génétique (fragments d'ADN génomique ou c-DNA, etc....) dans le tissu musculaire.

En particulier, il est possible d'utiliser lesdites séquences pour exprimer des séquences nucléotidiques ayant une fonction d'immunomodulateurs, par exemple des cytokines, ou des propriétés immunogènes, par exemple la protéine VP1 du poliovirus ou HBs du virus de l'hépatite B.

Une autre application se trouve dans le domaine de la thérapie génique. En particulier, et de manière générale les techniques nécessitant l'introduction d'ADN dans des tissus nécessitent l'utilisation de séquences nucléotidiques, ou de vecteurs les contenant, ou de liposomes, exprimant de manière importante les gènes ou ADNc portés par ces séquences. A ce jour ce problème n'a pas été résolu de manière satisfaisante.

Le demandeur s'est donc attaché à déterminer quelles parties des séquences régulatrices du gène de la desmine étaient responsables de l'activité amplificatrice.

Il a ainsi trouvé de manière surprenante le rôle joué par une séquence comprise entre les sites MEF2 et MyoDl dans l'activité amplificatrice.

Il a aussi mis en évidence que le couplage d'une telle séquence amplificatrice avec un promoteur permettait l'expression de gènes ou d'ADNc codant pour des peptides, des polypeptides, ou des protéines d'intérêt thérapeutique par introduction directe de l'ADN.

La présente invention a pour objet une séquence amplificatrice présentant une homologie d'au moins 90% avec la séquence SEQ ID N'1 suivante: dans laquelle:
- X₁, X₂ et X₃ peuvent représenter indépendamment respectivement C ou G, C ou G, et C ou A,
- Y₁, Y₂ et Y₃ peuvent représenter indépendamment respectivement T ou C, C ou G, et T ou C.

Avantageusement une telle séquence s'hybride en conditions stringentes, c'est-à-dire en conditions rigoureuses, avec la séquence SEQ ID N°1.

De telles conditions sont par exemple les suivantes:
Hybridation à 65°C pendant la nuit:
tampon:
0,2 % polyvinyl pyrrolidone
0,2% ficoll 400
0,2% Sérum Albumine Bovine (BSA)
2XSSC
100 µg/ml d'ADN de sperme de saumon
150 µg/ml d'ARN de levure
Lavages:
2xSSC 10 minutes à 65°C une fois
2xSSC 30 minutes à 55°C 4 fois

Un autre objet de la présente invention est une séquence amplificatrice présentant au moins 90% d'homologie avec la séquence SEQ ID N°2 suivante: dans laquelle Z peut être T ou C.

Avantageusement une telle séquence s'hybride en conditions stringentes, ou rigoureuses, avec la séquence SEQ ID N°2.

On notera que la présente invention couvre toutes les séquences amplificatrices différant par un ou plusieurs nucléotides par rapport aux séquences SEQ ID N°1 et SEQ ID N°2 et dont le fonctionnement n'est pas modifié de manière sensible ainsi que leurs séquences complémentaires.

On entend dans la présente demande par séquence amplificatrice une séquence amplifiant très fortement la transcription d'un ou plusieurs gènes situés en cis.

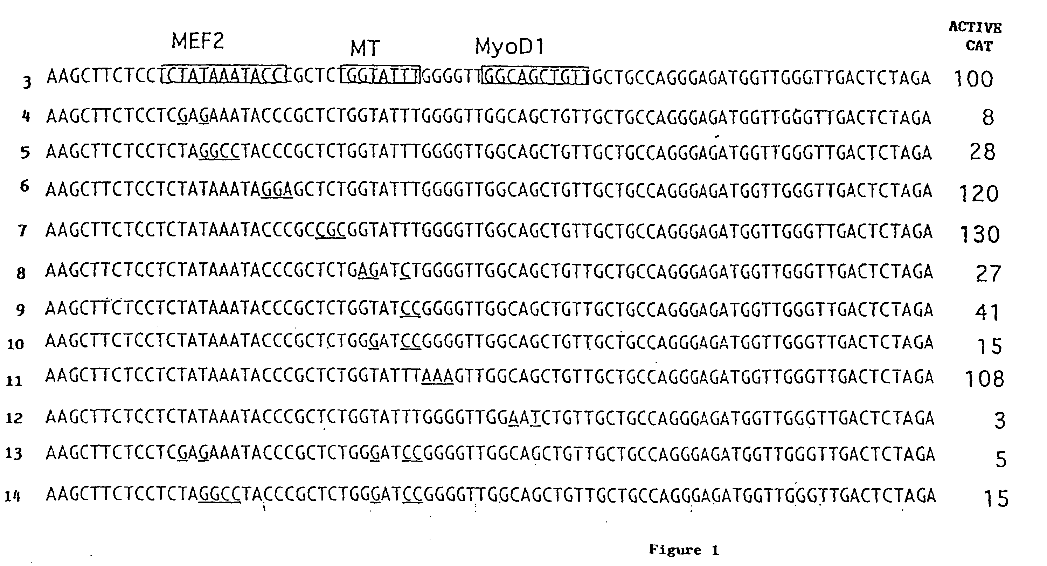

Font aussi partie des séquences selon l'invention, la séquence n°3 ou la séquence n°6, ou la séquence n°7 ou la séquence n°11, telles que définies par référence à la figure 1, seules ou sous forme de polymères de deux à huit fragments identiques ou associés à l'une des quatre séquences.

Le terme "vecteur" est quant à lui utilisé pour désigner une molécule d'acide nucléique dans laquelle il est possible d'insérer des fragments d'acide nucléique étranger, pour ensuite les introduire et les maintenir dans une cellule hôte.

Le terme liposome concerne toute composition lipidique capable de fixer de l'ARN ou de l'ADN de manière covalente ou non-covalente.

La présente invention est en outre relative à une séquence ou un vecteur d'expression contenant ladite séquence et au moins:
- une séquence, du type SEQ ID N° 1 ou SEQ ID N°2 telles que décrites ci-dessus, en une ou plusieurs copies, à l'exception des séquences dans lesquelles X₁, X₂, X₃ et Y₂ représentent une cytosine (C) et Y₁ et Y₃ représentent une thymidine (T) ou Z représente (T), auquel cas les séquences SEQ ID N°1 ou SEQ ID N°2 sont en au moins deux copies, et
- une séquence codant pour une protéine ou correspondant à la séquence génomique codant pour ladite protéine ou partie de celle-ci.

Avantageusement une telle séquence, ou un tel vecteur, est complété par un, ou plusieurs, promoteur (s) permettant l'expression de ladite protéine dans des cellules transfectées par ledit ADN.

La protéine peut être au moins une partie d'un antigène bactérien par exemple l'uréase de H. pylori, dite HSP (A) en abréviation de "Heat-schock protein A", ou la protéine HSP (B), ou viral, par exemple l'antigène de surface du virus d'une hépatite en particulier de l'hépatite B, et préférentiellement la protéine HBs sous l'une de ses formes S, S-préS2 ou S-préS2-préS1. Elle peut aussi être une protéine d'un virus d'une hépatite A, ou d'une hépatite non-A, non-B, telle qu'une hépatite C, E ou delta.

Les séquences des gènes ou des protéines des bactéries ou virus de ces hépatites sont décrites ou peuvent être déduites des documents suivants : brevet FR-79 21 811,(publié sous le n' 2.464.269) , FR-80.09.039, (publié sous le N° 2.480.779) ( EP-81.400.634 (publié sous le n° 0 038 765) FR-84.03.564,(publié sous le n° 2 560 890), EP-91.830.479 (publié sous le n° 0 485 347) et FR 88.13.135 (publication n° 2.637.612), demande PCT publiée sous le n° WO 94/26.901 et article de Najarian et al. (Proc. Natl. Acad. Sci. USA, 1985, 82, 2627-2631).

La protéine peut aussi être une protéine ou une partie d'une protéine du virus HIV-1, du virus HIV-2, ou du virus HTLV-1, en particulier la protéine ENV (correspondant à l'enveloppe virale) ou la protéine gag. La séquence de la partie de la protéine, ou du peptide, est choisie en fonction de la fonction que l'on cherche à exprimer. Les séquences nucléotidiques minimales sont avantageusement comprises entre 20 et 50 paires de bases.

Dans le cas du virus HTLV-1, les protéines ou les séquences codant pour ces protéines peuvent être celles décrites dans les documents suivants, ou peuvent être déduites de ces documents : demandes PCT/WO-93/06 843, PCT/WO 90 15 820, EP-0 352 060, et EP-0 269 445, et articles de Gray et al. (1990, Virology, 177, 391-395), Tanaka et al. (1991, J. Immunol., 147, 354-360) et Nakamura et al. (1987, Int. J. Cancer, 40,403-407).

De manière générale, ladite protéine peut être une ou plusieurs des protéines présentant un intérêt thérapeutique ou vaccinal, telles que des protéines d'intérêt immunothérapeutique, par exemple les Interleukines, les facteurs de croissance, par exemple les facteurs de croissance des fibroblastes (FGF) ou le N G F (Nerve Growth Factor) ou le gène maltase, des protéines pouvant induire une réponse immunitaire, que ce soit une immunité humorale, cytotoxique ou cellulaire, ou des protéines permettant de complémenter l'activité d'un gène normalement exprimé chez l'individu à traiter mais qui ne l'est plus, soit par mutation ou délétion de sa séquence.

Ladite protéine peut aussi être une protéine hybride, constituée par exemple d'au moins une partie de la desmine et d'au moins une partie d'une protéine dont on cherche à compenser la déficience. Dans ce cas la séquence, ou le vecteur, peut comprendre une séquence activatrice, ou amplificatrice du gène de la desmine, une séquence codant pour au moins une partie de la desmine et une séquence codant pour la protéine dont on cherche à compenser la déficience.

On notera que les séquences d'amplification peuvent être situées indifféremment en amont ou en aval de la séquence codant pour une protéine, ou un fragment de celle-ci, ou pour un peptide compris entre 10 et 50 acides aminés, et de son promoteur, et être dans les deux orientations possibles.

Outre une séquence codant pour une protéine ou un fragment de celle-ci et une séquence amplificatrice, ladite séquence, ou vecteur, peut aussi comprendre d'autres séquences de régulation de type Krox, telles que la séquence Krox 24 (LEMAIRE, P. et al. 1990, Mol. Cell. Biol, 10, 3456-3467).

Le promoteur peut être tout promoteur seul ou associé à un autre promoteur permettant l'expression de la protéine telle que définie ci-dessus. Il peut ainsi être un promoteur interne au gène à exprimer ou à l'ADNc codant pour la protéine ou tout fragment de celle-ci à exprimer. Il peut aussi être un promoteur homologue à l'hôte, tel que le promoteur d'un gène d'une protéine du cytosquelette, en particulier celui de la desmine tel que décrit par BOLMONT et al. (Journal of submicroscopic cytology and pathology, 1990, 22, 117-122) ou LI et al. (Gene, 1989, 78, 243-254).

Il peut encore être un promoteur hétérologue, tel que celui d'un virus, par exemple celui de la thymidine kinase du virus de l'herpès (HSV).

Outre le promoteur, situé en amont de la séquence codant pour la protéine ou pour tout fragment de celle-ci, la séquence peut aussi comprendre une séquence de terminaison de la transcription située en aval de la séquence codant pour la protéine.

Enfin, une telle séquence, ou un tel vecteur peut comprendre des séquences permettant la recombinaison homologue chez l'organisme traité, spécifique du gène à remplacer, lesdites séquences étant placées en amont et en aval de la séquence codant pour une protéine, et éventuellement des séquences activatrices naturelles et/ou du ou des promoteur(s). Du fait de la présence de telles séquences, le gène non désiré, présent dans l'organisme traité sera remplacé par le gène porté par le vecteur ou la séquence, et que l'on désire voir s'exprimer dans l'organisme.

Une telle méthode de recombinaison homologue peut être du type de celle décrite par Le Mouellic et al. (1990, Proc. Natl. Acad. Sci. USA, 87, 4712-4716), ou dans la demande PCT WO 91/06.667.

Un vecteur tel que défini ci-dessus peut comprendre en outre une origine de réplication active dans un microorganisme, tel qu'une bactérie.

Un tel vecteur peut aussi comprendre un gène permettant sa sélection dans ledit microorganisme, tel qu'un gène de résistance à un antibiotique. Il peut être un plasmide.

Avantageusement, un tel microorganisme est Escherichia coli.

Les vecteurs d'expression contenant les séquences à exprimer et les séquences transfectables, objets de la présente invention peuvent être obtenus par les méthodes connues de l'homme du métier, en particulier par synthèse chimique, telle que la méthode commercialisée par Applied Biosystem, ou par les méthodes du génie génétique. De telles méthodes sont celles décrites en particulier dans Maniatis T. et al. 1982 - Molecular Cloning, A Laboratory Manual, Cold Spring Harbor - Ed. New York, ou l'une de ses récentes rééditions.

L'invention est encore relative à deux plasmides dont les références d'identification sont respectivement E03D CAT et DMT tk CAT (voir Exemples 1 et 2 ci-après) et qui ont été déposés à la collection CNCM de l'Institut Pasteur le 16 Décembre 1994 sous les numéros d'enregistrement respectifs I-1508 et I-1509.

La présente invention a en outre pour objet une composition pharmaceutique comprenant une quantité pharmacologiquement efficace d'un des vecteurs d'expression ou d'une des séquences nucléotidiques comportant au moins l'une des séquences activatrices selon l'invention décrits ci-dessus exprimables sans vecteur dans les cellules à transfecter.

Une telle composition peut aussi comprendre des excipients pharmaceutiquement compatibles.

La présente invention est encore relative à un médicament ou un vaccin contenant ces séquences et vecteurs.

Bien que l'invention ne soit pas limitée à une thérapie précise, les vecteurs et séquences sont préférentiellement destinés à être utilisés dans un but vaccinal, et particulièrement à être injectés dans des tissus musculaires. Dans un tel mode de mise en oeuvre de l'invention, le promoteur choisi est avantageusement celui d'un gène ou d'un ADNc codant une protéine musculaire ou un fragment de celle-ci.

La présente invention est illustrée sans pour autant être limitée par les exemples qui suivent.

La figure 1 illustre l'effet de mutation dans la séquence amplificatrice du vecteur DMT tk CAT sur l'activité du gène codant pour la CAT qu'il porte.

La figure 2 est un autoradiogramme d'un gel d'extraits cellulaires des lignées C 2,7 (puits C2) et CCL 136 (puits Rd), incubés avec l'oligonucléotide MT marqué.La position indiquée par le chiffre 1 correspond au complexe MT-protéine.

La figure 3 est un autoradiogramme d'un filtre obtenu par hybridation de type Southwestern entre des extraits cellulaires des lignées C 2,7 et CCL 136 (puits RD) et l'oligonucléotide MT marqué. Des marqueurs de poids moléculaires (Sigma) ont été mis à migrer à partir du puits MW.

### EXEMPLE 1:

### Préparation du vecteur d'expression D MT tk CAT:

En utilisant deux amorces, (1) DesHindIII ou (2) DesXbaI un fragment de 84 paires de bases qui contient un site de fixation (GGCAGCTGTT) pour le facteur transactivateur MyoDl, un site de fixation (CTATAAATACC) pour le facteur MEF2 et un site (GGTATTT) appelé MT a été amplifié à partir du promoteur du gène desmine humain. Ce fragment de 84 bp a été inséré dans les sites de restriction HindIII-XbaI du plasmide pBLCAT2 (Lucknow et Schütz, 1987, Nucl. Acid. Res., 15, 5490) qui contient le promoteur basal du gène thymidine kinase du virus herpès simplex et la région codante du gène bactérien chloramphénicol acétyltransférase (CAT).

Grâce à la coopération du site MT avec les sites MyoD1 et MEF2, ce fragment de 84 bp augmente 60 fois l'expression du gène indicateur CAT dans les cellules musculaires différenciées, les myotubes.

### EXEMPLE 2:

### Préparation du vecteur d'expression EO3 D CAT:

Un fragment de 280 paires de bases (-973 à - 693) qui contient l'amplificateur du gène desmine humain a été placé devant la région de 228 bp (Li et Paulin 1991, 1993 précédemment cités) qui contient le promoteur basal du gène desmine humain et la région codante du gène bactérien chloramphénicol acétyltransférase (CAT).

Un fragment de 280 paires de bases (-973 à - 693) qui contient l'amplificateur du gène desmine humain a été rendu bout-franc par l'ADN polymérase (Klenow) et inséré dans le site de restriction Hind III qui a été aussi rendu bout-franc par l'ADN polymérase (Klenow).

Cet amplificateur fonctionne non seulement dans les cellules musculaires différenciées, mais aussi dans les cellules musculaires mononucléées, les myoblastes. Ce fragment active 100 fois l'expression du gène dans les myotubes et 10 fois dans les myoblastes.

### EXEMPLE 3:

### Effets de mutations dans la région amplificatrice du gène desmine sur l'activité d'un gène rapporteur dans les myotubes.

Le vecteur DMT tk CAT dont la préparation a été décrite dans l'exemple 1 a été muté par la technique de mutagénèse dirigée (Kunkel et al. 1985, PNAS, 82, p.488-492). Ces vecteurs mutés ont été transfectés dans des myoblastes en culture et l'activité CAT a été mise en évidence, de la manière décrite par Li et al. (1991 et 1993 précédemment cités).

Les résultats de ces expérimentations sont résumés sur la figure . Onze constructions numérotées de 4 à 14 présentant diverses mutations soulignées ont été testés quant à leur effet sur l'activité CAT. La séquence amplificatrice non mutée est celle représentée en position 3.

Il apparaît très clairement que les mutations des constructions 6 et 7 entraînent une suramplification de l'activité CAT par rapport à la construction n°3.

Les deux vecteurs d'expression des exemples 1 et 2 peuvent être utilisés pour construire des vecteurs pour exprimer le gène d'intérêt thérapeutique dans les cellules musculaires en remplaçant le gène CAT par le gène choisi. Les sites EcoRI et BamH I peuvent être utilisés pour enlever le gène CAT.

Le gène CAT peut ainsi être remplacé pour l'immunisation ou la vaccination des mammifères avec de l'ADN nu ou de l'ADN génomique correspondant aux gènes qui codent l'enveloppe et la protéine gag du virus HTLV1 ( Gary et al., 1990; Virology 177, 391-395; Tanaka et al., 1991, J. Immunology 147: 354-360; Nakamura et al. 1987; Int. J.Cancer 40: 403-407); le gène codant tout ou partie de l'enveloppe du virus hépatite B ou des papillomes humains tels que HPV16, HPV33, etc....; le gène qui code pour l'enveloppe du FIV, ou pour la thérapie génique , les gènes qui codent les interleukines et les facteurs de croissance (FGF, NGF, MG-CSF etc...) et dans le cadre d'une thérapie génique pour une myopathie due à une déficience en maltase.

### EXEMPLE 4:

### Mise en évidence d'une protéine se fixant spécifiquement sur la séquence MT (GGTATTT).

### Matériel et Méthodes

### 1) Retard sur gel

Des extraits nucléaires issus des lignées musculaires C 2,7 (Li et Paulin, 1991, J. Biol. Chem., 266, 6562-6570) et Rhabdomyosarcoma (accessible à l'ATCC sous le n° CCL 136) sont incubés avec l'oligonucléotide MT marqué à la radioactivité à température ambiante pendant 10 minutes. Les mélanges sont ensuite mis à migrer sur un gel d'acrylamide à 5%. Après 3 heures de migration, le gel est séché et autoradiographié.

Les mélanges sont réalisés dans un volume de 20µl contenant 750 ng d'ADN de sperme de saumon soniqué, 3 µg d'extrait nucléaire, 15 mM HEPES pH 8, 0,1 mM EDTA, 1 mM DTT, 2 mM MgCl₂, 70 mM NaCl, 15% glycérol.

### 2) Hybridation de type Southwestern.

Des extraits nucléaires sont préparés à partir des lignées musculaires C 2,7 et Rhabdomyosarcoma. 40µg de protéines sont mis à migrer sur un gel SDS-PAGE à 10%. Les protéines sont transférées sur un filtre de nitrocellulose. Le filtre est incubé pendant une heure à 37°C dans une solution contenant 5% de lait écrèmé; 50 mM Tris pH 7,5; 50 mM NaCl; 0,1 mM EDTA, 1 mM DTT puis dans un tampon contenant la sonde radioactive MT. Le filtre est lavé trois fois dans le même tampon et autoradiographié.

### Résultats.

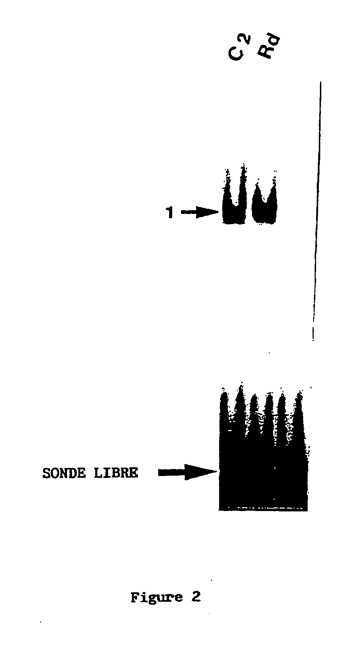

Les expériences de retard sur gel (figure 2) montrent que la séquence MT (GGTATTT) est capable de fixer spécifiquement un facteur nucléaire issu des lignées musculaires murine (C 2,7) et humaine (Rhabdomyosarcoma).

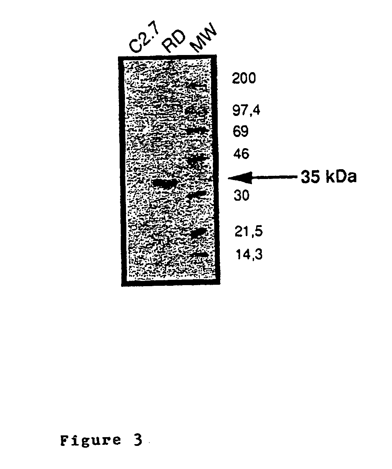

L'autoradiogramme obtenu par la méthode d'hybridation Southwestern (figure 3) montre que le facteur nucléaire qui se fixe sur la séquence MT est une protéine de 35 kDa.

Cette protéine est présente dans les cellules musculaires telles que les cellules C 2,7 et Rhabdomyosarcoma.

La quantité de cette protéine augmente au cours de la différenciation musculaire.

## Revendications

1. Séquence amplificatrice présentant une homologie d'au moins 90% avec la séquence SEQ ID N° 1 suivante: dans laquelle :
- X₁, X₂ et X₃ peuvent représenter indépendamment respectivement C ou G, C ou G, et C ou A,
- Y₁, Y₂ et Y₃ peuvent représenter indépendamment respectivement T ou C, C ou G, et T ou C.

2. Séquence selon la revendication 1 **caractérisée en ce qu'**elle s'hybride en conditions stringentes, ou rigoureuses, avec la séquence SEQ ID N°1.

3. Séquence amplificatrice présentant une homologie d'au moins 90% avec la séquence SEQ ID N°2 suivante : dans laquelle Z peut être T ou C.

4. Séquence selon la revendication 3
**caractérisée en ce qu'**elle s'hybride en conditions stringentes, ou rigoureuses, avec la séquence SEQ ID N°2.

5. Séquence comprenant au moins :
- une séquence d'amplification selon l'une des revendications 1 à 4, en une ou plusieurs copies, à l'exception des séquences dans lesquelles X₁, X₂, X₃ et Y₂ représentent une cytosine (C) et Y₁ et Y₃ représentent une thymidine (T) ou Z représente (T), auquel cas les séquences SEQ ID N°1 ou SEQ ID N°2 sont en au moins deux copies, et
- une séquence codant pour une protéine ou correspondant à la séquence génomique codant pour ladite protéine ou partie de celle-ci.

6. Séquence complémentaire d'une séquence selon l'une des revendications 1 à 5.

7. Séquence selon la revendication 5 **caractérisée en ce qu'**elle comprend de plus un ou plusieurs promoteur(s) permettant l'expression de ladite protéine, dans des cellules transfectées par ledit ADN.

8. Séquence selon la revendication 5 **caractérisée en ce qu'**elle comprend un promoteur interne au gène ou à l'ADNc.

9. Séquence selon la revendication 5 **caractérisée en ce qu'**elle comprend un promoteur hétérologue au gène.

10. Séquence selon l'une des revendications 5 et 7 **caractérisée en ce qu'**elle comprend des séquences permettant la recombinaison homologue d'un gène à remplacer chez un organisme, lesdites séquences étant placées en amont et en aval de la séquence codant pour une protéine, et éventuellement du promoteur.

11. Séquence selon la revendication 5, **caractérisée en ce qu'**elle porte une séquence de type Krox.

12. Séquence selon l'une des revendications 7 et 9, **caractérisée en ce que** ledit promoteur est celui du gène de la desmine ou de la thymidine kinase.

13. séquence selon l'une des revendications 5 et 7 à 12 , **caractérisée en ce que** la protéine codée est une protéine présentant des propriétés thérapeutiques ou vaccinales.

14. séquence selon l'une des revendications 5 et 7 à 12, **caractérisée en ce que** la protéine codée est au moins une partie d'un antigène bactérien par exemple l'uréase, la protéine de H. pylori dite HSP(A), ou la protéine HSP (B), ou viral, par exemple l'antigène de surface du virus d'une hépatite, en particulier l'hépatite B, et préférentiellement la protéine HBs, d'une hépatite A ou d'une hépatite non-A non-B, telle qu'une hépatite C, E ou Delta, ou encore une partie d'une protéine du virus HIV-1, du virus HIV-2, ou du virus HTLV-1, en particulier la protéine ENV (correspondant à l'enveloppe virale) ou la protéine gag, et de manière générale, une protéine d'intérêt immunothérapeutique, par exemple les interleukines, les facteurs de croissance, par exemple les facteurs de croissance des fibroblastes (FGF) ou des nerfs (NGF), des protéines pouvant induire une réponse immunitaire, humorale ou cytotoxique, ou des protéines permettant de complémenter l'activité d'un gène normalement exprimé chez l'individu à traiter mais qui ne l'est plus soit par mutation ou délétion de sa séquence tel que le gène maltase.

15. vecteur comprenant une séquence selon l'une des revendications 5 à 14 .

16. Vecteur selon la revendication 15 **caractérisé en ce qu'**il comprend une origine de réplication active dans un microorganisme.

17. Vecteur selon l'une des revendications 15 et 16, **caractérisé en ce qu'**il porte une origine de réplication active dans Escherichia coli.

18. Vecteur selon l'une des revendications 15 à 17, **caractérisé en ce qu'**il porte un gène de résistance s'exprimant dans un microorganisme.

19. vecteur selon l'une des revendications 15 à 18, **caractérisé en ce qu'**il est un plasmide.

20. Microorganisme portant une séquence ou un vecteur selon l'une des revendications 5 à 18.

21. Microorganisme selon la revendication 20, tel que déposé et enregistré à la CNCM sous le n° I-1508 ou I-1509.

22. Composition pharmaceutique **caractérisée en ce qu'**elle comprend une quantité pharmacologiquement efficace d'une séquence ou d'un vecteur selon l'une des revendications 5 à 18.

23. Composition selon la revendication 22, **caractérisée en ce qu'**elle comprend des excipients pharmacologiquement compatibles.

24. Médicament ou vaccin **caractérisé en ce**
**qu'**il comprend un vecteur ou une séquence selon l'une des revendications 5 à 18.

25. Utilisation de la composition selon l'une des revendications 22 ou 23 ou du médicament selon la revendication 24 pour l'obtention d'un médicament destiné à traitement médical pour l'expression des séquences nucléotidiques ayant une fonction d'immunomodulateurs, par exemple cytokines ou des propriétés immunogènes, par exemple protéine VP1 du poliovirus ou HBs de l'hépatite B ainsi que pour l'obtention d'un médicament destiné à la thérapie génique par introduction d'ADN dans des tissus nécessitant l'utilisation des séquences ou des vecteurs exprimant de manière importante les gènes portés par ces séquences.

## Patentansprüche

1. Amplifikationssequenz, die eine Homologie von wenigstens 90% mit der folgenden Sequenz SEQ ID Nr. 1 aufweist: wobei:
- X₁, X₂ und X₃ unabhängig voneinander C oder G, C oder G bzw. C oder A darstellen können;
- Y₁, Y₂ und Y₃ unabhängig voneinander T oder C, C oder G bzw. T oder C darstellen können.

2. Sequenz gemäß Anspruch 1, **dadurch gekennzeichnet, dass** sie unter stringenten bzw. rigorosen Bedingungen mit der Sequenz SEQ ID Nr. 1 hybridisiert.

3. Amplifikationssequenz, die eine Homologie von wenigstens 90% mit der folgenden Sequenz SEQ ID Nr. 2 aufweist: wobei Z = T oder C sein kann.

4. Sequenz gemäß Anspruch 3, **dadurch gekennzeichnet, dass** sie unter stringenten bzw. rigorosen Bedingungen mit der Sequenz SEQ ID Nr. 2 hybridisiert.

5. Sequenz, die wenigstens folgendes umfasst:
- eine Amplifikationssequenz gemäß einem der Ansprüche 1 bis 4 in einer oder mehreren Kopien, mit Ausnahme der Sequenzen, bei denen X₁, X₂, X₃ und Y₂ ein Cytosin (C) darstellen und Y₁ und Y₃ ein Thymidin (T) darstellen oder Z (T) darstellt, in welchem Fall die Sequenzen SEQ ID Nr. 1 oder SEQ ID Nr. 2 in wenigstens zwei Kopien vorliegen; und
- eine Sequenz, die für ein Protein codiert oder der genomischen Sequenz entspricht, die für das Protein oder einen Teil davon codiert.

6. Sequenz, die einer Sequenz gemäß einem der Ansprüche 1 bis 5 komplementär ist.

7. Sequenz gemäß Anspruch 5, **dadurch gekennzeichnet, dass** sie außerdem einen oder mehrere Promotoren umfasst, die die Expression des Proteins in mit der DNA transfizierten Zellen erlauben.

8. Sequenz gemäß Anspruch 5, **dadurch gekennzeichnet, dass** sie einen internen Promotor des Gens oder der cDNA umfasst.

9. Sequenz gemäß Anspruch 5, **dadurch gekennzeichnet, dass** sie einen zu dem Gen heterologen Promotor umfasst.

10. Sequenz gemäß einem der Ansprüche 5 und 7, **dadurch gekennzeichnet, dass** sie Sequenzen umfasst, die die homologe Rekombination eines zu ersetzenden Gens bei einem Organismus erlauben, wobei die Sequenzen stromaufwärts und stromabwärts der für ein Protein codierenden Sequenz und gegebenenfalls des Promotors gesetzt werden.

11. Sequenz gemäß Anspruch 5, **dadurch gekennzeichnet, dass** sie eine Sequenz des Krox-Typs trägt.

12. Sequenz gemäß einem der Ansprüche 7 und 9, **dadurch gekennzeichnet, dass** der Promotor derjenige des Gens von Desmin oder der Thymidin-Kinase ist.

13. Sequenz gemäß einem der Ansprüche 5 und 7 bis 12, **dadurch gekennzeichnet, dass** das codierte Protein ein Protein ist, das therapeutische oder Impfstoffeigenschaften aufweist.

14. Sequenz gemäß einem der Ansprüche 5 und 7 bis 12, **dadurch gekennzeichnet, dass** es sich bei dem codierten Protein um wenigstens folgendes handelt: ein Teil eines bakteriellen Antigens, zum Beispiel Urease, das HSP(A)-Protein von *H. pylori* oder das HSP(B)-Protein, oder eines viralen Antigens, zum Beispiel Oberflächen-Antigen eines Hepatitisvirus, insbesondere Hepatitis-B-Virus, und vorzugsweise das HBs-Protein, eines Hepatitis-A-Virus oder eines Nicht-A-nicht-B-Hepatitisvirus, wie Hepatitis-C-, -E- oder -Delta-Virus, oder auch ein Teil eines Proteins des HIV-1-Virus, des HIV-2-Virus oder des HTLV-1-Virus, insbesondere das Env-Protein (das der Virushülle entspricht) oder das Gag-Protein, und allgemein ein Protein von immuntherapeutischem Interesse, zum Beispiel Interleukine, Wachstumsfaktoren, zum Beispiel Fibroblasten- (FGF) oder Nerven-Wachstumsfaktoren (NGF), Proteine, die eine humorale oder cytotoxische Immunantwort induzieren können, oder Proteine, die die Aktivität eines Gens ergänzen können, das bei dem zu behandelnden Individuum normalerweise exprimiert wird, aber jetzt nicht mehr exprimiert wird, entweder durch Mutation oder Deletion seiner Sequenz, wie das Maltase-Gen.

15. Vektor, der eine Sequenz gemäß einem der Ansprüche 5 bis 14 umfasst.

16. Vektor gemäß Anspruch 15, **dadurch gekennzeichnet, dass** er einen Replikationsstartpunkt umfasst, der in einem Mikroorganismus aktiv ist.

17. Vektor gemäß einem der Ansprüche 15 und 16, **dadurch gekennzeichnet, dass** er einen Replikationsstartpunkt trägt, der in *Escherichia coli* aktiv ist.

18. Vektor gemäß einem der Ansprüche 15 bis 17, **dadurch gekennzeichnet, dass** er ein Resistenzgen trägt, das in einem Mikroorganismus exprimiert wird.

19. Vektor gemäß einem der Ansprüche 15 bis 18, **dadurch gekennzeichnet, dass** es sich um ein Plasmid handelt.

20. Mikroorganismus, der eine Sequenz oder einen Vektor gemäß einem der Ansprüche 5 bis 18 trägt.

21. Mikroorganismus gemäß Anspruch 20, wie er beim CNCM unter der Nr. I-1508 oder I-1509 hinterlegt und eingetragen ist.

22. Pharmazeutische Zusammensetzung, **dadurch gekennzeichnet, dass** sie eine pharmakologisch wirksame Menge einer Sequenz oder eines Vektors gemäß einem der Ansprüche 5 bis 18 umfasst.

23. Zusammensetzung gemäß Anspruch 22, **dadurch gekennzeichnet, dass** sie pharmakologisch annehmbare Arzneimittelhilfsstoffe umfasst.

24. Medikament oder Impfstoff, **dadurch gekennzeichnet, dass** es einen Vektor oder eine Sequenz gemäß einem der Ansprüche 5 bis 18 umfasst.

25. Verwendung der Zusammensetzung gemäß einem der Ansprüche 22 oder 23 oder des Medikaments gemäß Anspruch 24 zur Gewinnung eines Medikaments zur medizinischen Behandlung für die Expression von Nucleotidsequenzen mit immunmodulatorischer Funktion, zum Beispiel Cytokinen, oder immunogenen Eigenschaften, zum Beispiel VP1-Protein des Poliovirus oder HBs-Protein des Hepatitis-B-Virus, sowie zur Gewinnung eines Medikaments für die Gentherapie durch Einführung von DNA in Gewebe, die die Verwendung von Sequenzen oder Vektoren benötigen, die die in diesen Sequenzen enthaltenen Gene in hohem Maße exprimieren.

## Claims

1. Amplifying sequence showing a homology of at least 90 % with the following SEQ ID N°1 sequence : in which:
- X₁, X₂ and X₃ may independently represent respectively C or G, C or G, and C or A,
- Y₁, Y₂ and Y₃ may independently represent respectively T or C, C or G, and T or C,

2. Sequence according to claim 1, **characterized in that** it hybridizes under stringent conditions with sequence SEQ ID N°1.

3. Amplifying sequence having a homology of at least 90 % with the following SEQ ID N°2 sequence : in which Z may be T or C.

4. Sequence according to claim 3, **characterized in that** it hybridizes under stringent conditions with sequence SEQ ID N°2.

5. Sequence comprising at least:
- an amplifying sequence according to any one of claims 1 to 4, in one or more copies, with the exception of sequences in which X₁, X₂, X₃ and Y₂ represent a cytosine (C) and Y₁ and Y₃ represent a thymidine (T) or Z represents (T), in which case sequences SEQ ID N°1 or SEQ ID N°2 are at least two copies thereof, and
- a sequence coding for a protein or corresponding to the genomic sequence encoding said protein or part thereof.

6. Sequence which is complementary of a sequence according to one of claims 1 to 5.

7. Sequence according to claim 5, **characterized in that** it comprises,
moreover, one or more promoter(s) allowing the expression of said protein in cells transfected by said DNA.

8. Sequence according to claim 5, **characterized in that** it comprises a promoter internal to the gene or to the cDNA.

9. Sequence according to claim 5, **characterized in that** it comprises a promoter that is hetorologous to the gene.

10. Sequence according to any one of claims 5 and 7, **characterized in that** it comprises sequences allowing the homologous recombination of a gene to be replaced in an organism, said sequences being placed upstream and downstream from the sequence coding for a protein, and possibly from the promoter.

11. Sequence according to claim 5, **characterized in that** it comprises a Krox type sequence.

12. Sequence according to any one of claims 7 and 9, **characterized in that** said promoter is that of the desmine gene or thymidine kinase.

13. Sequence according to any one of claims 5 and 7 to 12, **characterized in that** the encoded protein is a protein having therapeutic or vaccine properties.

14. Sequence according to any one of claims 5 and 7 to 12, **characterized in that** the encoded protein is at least part of bacterial antigen, for example urease, the protein of H. pylori so-called HSP (A), or the HSP (B) protein, or of a viral antigen, for example the surface antigen of a hepatitis virus, in particular hepatitis-B, and preferably protein HBs, of a hepatitis-A or of a non-A non-B hepatitis, such as hepatitis C, E or delta, or even part of a protein of the HIV-1 virus, of the HIV-2 virus or of the HTLV-1 virus, in particular the ENV protein (corresponding to the virus envelope) or the gag protein, and generally a protein having immunotherapy interest, for example the Interleukins, the growth factors, for example, the growth factors of fibroblast (FGF) or nerve (NGF), proteins which are able to induce an immune, humoral or cytotoxic response, or proteins which allow complementary gene activity in a gene normally expressed in the individual to be treated but which is no longer expressed either through mutation or deletion of its sequence such as the maltase gene.

15. Vector comprising a sequence according to any one of claims 5 to 14.

16. Vector according to claim 15, **characterized in that** it comprises an active replication origin in a micro-organism.

17. Vector according to any one of claims 15 to 16, **characterized in that** it comprises an active replication origin in Escherichia coli.

18. Vector according to any one of claims 15 to 17, **characterized in that** it comprises a resistance gene expressing itself in a micro-organism.

19. Vector according to any one of claims 15 to 18, **characterized in that** it is a plasmid.

20. Micro-organism carrying a sequence or a vector according to any one of claims 5 to 18.

21. Micro-organism according to claim 20, such as filed and registered with the CNCM under n°I-1508 or I-1509.

22. Pharmaceutical composition, **characterized in that** it comprises a pharmacologically effective quantity of a sequence or of a vector according to any one of claims 5 to 18.

23. Composition according to claim 22, **characterized in that** it comprises pharmacologically compatible excipients.

24. Medicinal or vaccine product, **characterized in that** it comprises a vector or a sequence according to any one of claims 5 to 18.

25. Use of the composition according to any one of claims 22 and 23 or of the medicinal product according to claim 24 to obtain a medicinal product provided for a medical treatment for the expression of nucleotide sequences having an immunomodulatory function, for example cytokines, or immunogenic properties, for example the VPI protein of the poliovirus or HBS of hepatitis-B, and to obtain a medicinal product provided for gene therapy through the introduction of DNA into tissues requiring the use of sequences or vectors strongly expressing the genes carried by these sequences.


### LISTE DE SEQUENCES

(1) INFORMATIONS GENERALES:
   (i) DEPOSANT:
      (A) NOM: INSTITUT PASTEUR
      (B) RUE: 28 RUE DU DOCTEUR ROUX
      (C) VILLE: PARIS
      (E) PAYS: FRANCE
      (F) CODE POSTAL: 75724

      (A) NOM: UNIVERSITE PARIS 7
      (B) RUE: 2 PLACE JUSSIEU
      (C) VILLE: PARIS
      (E) PAYS: FRANCE
      (F) CODE POSTAL: 75251
   (ii) TITRE DE L' INVENTION: SEQUENCES AMPLIFICATRICES, VECTEURS PORTANT
      CES SEQUENCES ET LEURS UTILISATIONS DANS DES COMPOSITIONS POUR L'EXPRESSION DE SEQUENCES NUCLEOTIDIQUES DANS DES CELLULES TRANSFECTEES, APPLICATIONS THERAPE
   (iii) NOMBRE DE SEQUENCES: 2
   (iv) FORME DECHIFFRABLE PAR ORDINATEUR:
      (A) TYPE DE SUPPORT: Floppy disk
      (B) ORDINATEUR: IBM ?C compatible
      (C) SYSTEME D' EXPLCITATION: PC-DOS/MS-DOS
      (D) LOGICIEL: Patentln Release #1.0, Version #1.30 (OEB)
   (V) DONNEES DE LA DEMANDE ACTUELLE:
      NUMERO DE LA DEMANDE: WO PCT/WO96
   (vi) DONNEES DE LA DEMANDE ANTERIEURE:
      (A) NUMERO DE LA DEMANDE: FR 95 01 937
      (B) DATE DE DEPOT: 20-FEB-1995
(2) INFORMATIONS POUR LA SEQ ID NO: 1:
   (i) CARACTERISTIQUES DE LA SEQUENCE:
      (A) LONGUEUR: 41 paires de bases
      (B) TYPE: nucléotiqe
      (C) NOMBRE DE BRINS: double
      (D) CONFIGURATION: linéaire
   (ii) TYPE DE MOLECULE: ADN (génomique)
   (iii) HYPOTHETIQUE: NON
   (vi) ORIGINE:
      (A) ORGANISME: Homo sapiens
   (ix) CARACTERISTIQUE:
      (A) NOM/CLE: allele
      (B) EMPLACEMENT:replace(11, "g")
   (ix) CARACTERISTIQUE;
      (A) NOM/CLE: allele
      (B) EMPLACEMENT:replace(12, "g")
   (ix) CARACTERISTIQUE:
      (A) NOM/CLE: allele
      (B) EMPLACEMENT:replace(13, "a")
   (ix) CARACTERISTIQUE:
      (A) NOM/CLE: allele
      (B) EMPLACEMENT:replace(16, "c")
   (ix) CARACTERISTIQUE:
      (A) NOM/CLE: allele
      (B) EMPLACEMENT:replace(17, "g")
   (ix) CARACTERISTIQUE:
      (A) NOM/CLE: allele
      (B) EMPLACEMENT:replace(18, "c")
   (xi) DESCRIPTION DE LA SEQUENCE: SEQ ID NO: 1:
   TCTATAAATA CCCGCTCTGG TATTTGGGGT TGGCAGCTGT T 41
(2) INFORMATIONS POUR LA SEQ ID NO: 2:
   (i) CARACTERISTIQUES DE LA SEQUENCE:
      (A) LONGUEUR: 8 paires de bases
      (B) TYPE: nucléotide
      (C) NOMBRE DE BRINS: double
      (D) CONFIGURATION: linéaire
   (ii) TYPE DE MOLECULE: ADN (génomique)
   (iii) HYPOTHETIQUE: NON
   (vi) ORIGINE:
      (A) ORGANISME: Homo sapiens
   (ix) CARACTERISTIQUE:
      (A) NOM/CLE: alleie
      (B) EMPLACEMENT:replace (1, "c")
   (xi) DESCRIPTION DE LA SEQUENCE: SEQ ID NO: 2:
   TGGTATTT 8